# EUROPEAN PATENT SPECIFICATION

(11) **EP 1 418 416 B1**
(45) Date of publication and mention of the grant of the patent: **10.06.2009**
(21) Application number: 03025669.7
(22) Date of filing: 07.11.2003
(51) Int. Cl.: G01N 17/00, G01N 33/34

(54) **Process and apparatus for weatherability testing of images**
Verfahren und Vorrichtung zum Testen der Witterungsbeständigkeit von Bildern
Procédé et dispositif pour tester les effets des conditions environnementales sur des images

(30) Priority: 07.11.2002 JP 2002323799
(43) Date of publication of application: 12.05.2004
(73) Proprietor: CANON KABUSHIKI KAISHA, Tokyo (JP)
(72) Inventor: Ishikawa, Takayuki, Canon K.K., Tokyo (JP)
(74) Representative: Weser, Wolfgang

(56) References cited:
- WO-A-01/66647
- WO-A-02/062570
- JP-A- 2003 300 376
- PATENT ABSTRACTS OF JAPAN vol. 2000, no. 24, 11 May 2001 (2001-05-11) & JP 2001 194290 A (CANON INC), 19 July 2001 (2001-07-19)
- PATENT ABSTRACTS OF JAPAN vol. 2000, no. 24, 11 May 2001 (2001-05-11) & JP 2001 194291 A (CANON INC), 19 July 2001 (2001-07-19)
- PATENT ABSTRACTS OF JAPAN vol. 006, no. 245 (P-159), 3 December 1982 (1982-12-03) & JP 57 141537 A (TOKYO SHIBAURA DENKI KK), 1 September 1982 (1982-09-01)

## Description

### BACKGROUND OF THE INVENTION

### Field of the Invention

The present invention relates to a process and an apparatus for the weatherability test of images, and particularly to a process and an apparatus for weatherability test for evaluating the weatherability of images such as ink-jet images and silver halide photographic images that are sensitive to light and environmental gas.

### Related Background Art

In recent years, color printers of the type that inks of plural colors are ejected from a head have been widely spread as output apparatus for computers and have come to be widely used in printing images processed by a computer or the like as multi-color, variable-contrast and high-definition images. In particular, a high-definition color print image making use of exclusive recording paper for ink-jet and an exclusive ink set for photographic image recording has come to be provided as an extremely high-definition and high-saturation image comparable with a silver halide. photograph.

Under such circumstances, there is a demand for higher weatherability (image fastness properties to light and environmental gas), and various investigations are made. More specifically, it is investigated to develop materials for ink-jet recording that a high-quality image can be retained over a long period of time. In such researches and developments, the accelerated weatherability test is indispensable.

As apparatus for a light fastness test, there have heretofore been known testing apparatus that an image sample can be tested under various conditions by placing the sample in a test chamber having a holder capable of suitably fitting the sample and being able to control temperature and humidity and using a light source having a spectral distribution close to light to be simulated, for example, a lamp such as a xenon lamp for sunlight or a high luminance fluorescent lamp for indoor light (Japanese Patent Application Laid-Open No. H05-312713 and the like).

Japanese Patent Application Laid-Open No. S58-45539 describes an apparatus for environmental test equipped with means for controlling temperature, atmospheric pressure and the like of a test chamber.

In the conventional apparatus for weatherability test, however, a synergistic action between light and gas and detailed testing conditions have not been sufficiently investigated and hence have been unable to simulate weatherability in an actual environment. In particular, it has been very difficult to exactly simulate the weatherability of an ink-jet recorded image very sensitive to environmental conditions.

WO 02/062570 A discloses an adhesive sheet for image protection which can be attached to images for protection against abrasive wear and for preventing image discoloration over a long time period, as well as controlled aging of an image under predetermined temperature and humidity conditions using a predetermined exposure to ultraviolet light.

WO 01/66647 A discloses an ink for inkjet printing, the ink comprising chemical compounds particularly stable to oxidative gases such as ozone and nitrous oxides, as well as an ozone test cabinet for assessing the ozone stability of inkjet images.

A testing process according to the preamble of claim 1 is described in "Issues in Evaluation and Standardization of Light Fading Tests of Ink Jet Materials" by B. Vogt and F. Frey (Proceedings IS&T's NIP17, 2001, pages 218 - 221).

### SUMMARY OF THE INVENTION

It is an object of the present invention to provide a process for weatherability test of an image that can complexly and exactly simulate light fastness and gas fastness of an ink-jet recorded image or the like.

According to the present invention, there is thus provided the testing process claimed in claim 1.

The other claims relate to further developments.

### BRIEF DESCRIPTION OF THE DRAWING

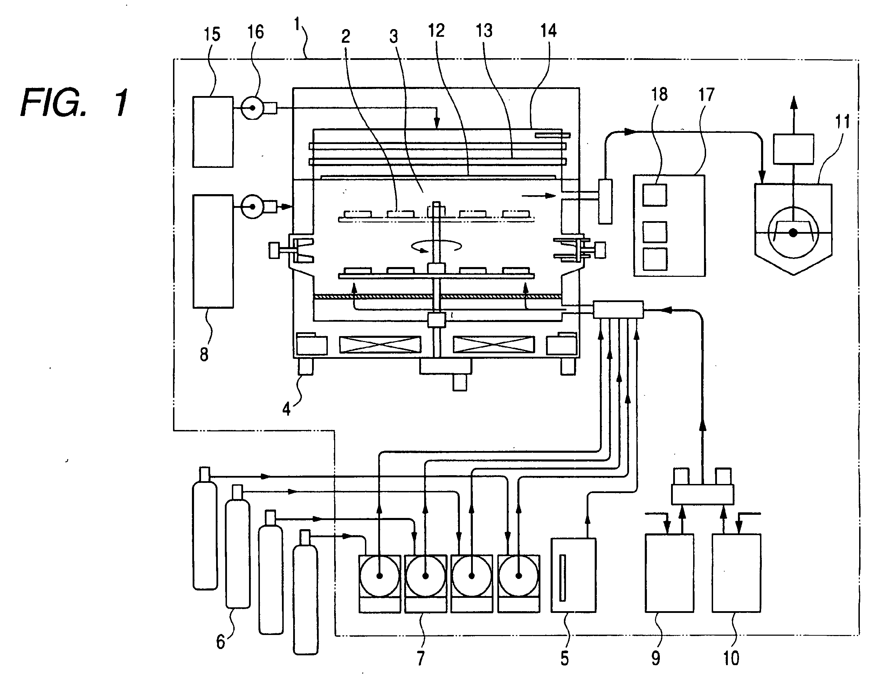

Fig. 1 schematically illustrates the construction of an apparatus used for weatherability test of an image according to the present invention:

### DESCRIPTION OF THE PREFERRED EMBODIMENTS

The mixed gas used in the present invention contains ozone and at least another gas than ozone. Preferable examples of said at least another gas than ozone include nitrogen dioxide, sulfur dioxide, hydrogen sulfide, ammonia, chlorine, hydrogen chloride and hydrogen fluoride. In the present invention, the composition of the mixed gas may preferably be set according to the environment to be simulated. However, it is particularly preferred to use a mixed gas of ozone, nitrogen dioxide, and sulfur dioxide. It is more preferably that hydrogen sulfide and chlorine are used in addition to these three gasses.

The reason why the use of the mixed gas makes the evaluated result more close to an actual environment is not clearly known. However, it is considered to be as follows.

The ink-jet ink is generally alkaline. Since this ink is applied to a recording medium being acidic, the resulting image tends to be affected by acid alkali characteristics of an environment. On the other hand, since the actual environment is weakly acid, it is considered that it is hard to exactly simulate the actual environment by using only a neutral gas such as ozone.

Proportions of these gasses mixed are 100 to 3,000 ppb for ozone, 100 to 3,000 ppb for nitrogen dioxide, 25 to 900 ppb for sulfur dioxide, 1 to 150 ppb for hydrogen sulfide and 1 to 30 ppb for chlorine, in terms of volume proportions. Among these, a ratio of ozone to nitrogen dioxide to sulfur dioxide is preferably 2:2:0.5 to 5:5:1.5, more preferably 4:4.1:1. A mixed gas of nitrogen and oxygen is preferably used as a diluent gas because this mixed gas does not adversely affect the image. The diluent gas is preferably obtained by passing air through a catalyst or the like to remove other components than nitrogen and oxygen because it is cheap.

These gasses may be supplied separately into a test chamber or mixed in advance and then supplied to the test chamber. As a method for supplying the mixed gas, the mixed gas of a prescribed compositional ratio may be continuously supplied with a constant flow rate to the test chamber during the weatherability test. Alternatively, the interior of the test chamber may be set to the mixed gas atmosphere, and the supply of the gas may be then stopped during the weatherability test. In the present invention, the weatherability characteristics of the image can be analyzed in detail by analyzing the compositional ratio of the gas after the weatherability test. When many samples are tested at the same time, the former method is preferred. Incidentally, a means for supplying the gas may be either a gas generator or a general gas cylinder.

In the present invention, the mixed gas may be changed three to five times per hour during the weatherability test. Specifically, the flow rate of the mixed gas per hour is preferably 500 to 5,000 liters, more preferably 1000 to 3,000 liters. When an image to be tested is an image obtained by absorbing an ink in pores of a recording medium like ink-jet recording, the flow velocity of the mixed gas is preferably 0.1 to 2.5 m/s, more preferably 0.5 to 2 m/s because the flow velocity may have an influence that the image to be tested is deteriorated.

The light source used is preferably suitably selected according to conditions intended to simulate, for example, outdoor or indoor environment. In the case of outdoor light (sunlight), a xenon lamp, a halogen lamp or the like is preferably used. The xenon lamp is particularly preferably used. In the case of indoor light, a fluorescent lamp, tungsten-filament lamp or the like is preferably used. The fluorescent lamp is particularly preferably used.

The quantity of light to be applied is also suitably selected according to the conditions intended to simulate. For example, intensity of light is set to be 1 × 10³ to 1 × 10⁵ lux, and the irradiation time is controlled, whereby the conditions to exactly simulate are preferably preset.

In the present invention, an influence on the temperature, humidity and gas concentration atmosphere within the test chamber is preferably reduced to the minimum. To do so, the light source is cooled. It is also preferred that temperature controlling means and humidity controlling means be provided within the test chamber. By providing these means, the weatherability of an image to be tested can be more exactly and simply simulated under a specific environment, for example, an environment that the temperature is 24°C and the relative humidity (RH) is 60%.

The temperature controlling means are preferably controlling means of the heat exchange type. The humidity controlling means are preferably humidifying means in which means for humidifying with steam obtained by heating water are provided at another place than the test chamber, and a water drop preventing mechanism for avoiding penetration of water drops caused by the steam into the test chamber is fitted. By providing such means, even an image obtained by using a material and a recording system sensitive to temperature and humidity, such as an image obtained by ink-jet recording may be subjected to the exact weatherability test.

The process for weatherability test of an image according to the present invention will hereinafter be described with reference to Fig. 1 that schematically illustrates the construction of an apparatus used for weatherability test of an image according to the present invention.

In Fig. 1, reference numeral 1 indicates the whole construction of the apparatus used for weatherability test of an image according to the present invention, 2 an image as a sample, and 3 a test chamber. To this test chamber 3 are connected a circulation blower 4 for circulating an atmosphere within the test chamber, an environmental gas generator 5, a standard gas cylinder 6, a constant displacement pump 7 for diluent gas for controlling the flow rate of the gas to introduce it into the test chamber, a circulation cooling unit 8 for cooling the gas stream within the apparatus that is temperature controlling means for test chamber, a humidifier 9 and a dehumidifier 10 for controlling the humidity of the gas stream within the apparatus, and an exhaust gas treating device 11 for discharging an exhaust gas to the air after completion of the test through ducts and pipes guiding the atmospheric stream.

Incidentally, a plurality of samples 2 may be set to a sample holder equipped with a rotating device. An agitating device for reagitating the atmosphere may also be suitably provided. To the humidifier 9, is given a structure for preventing penetration of water drops, such as prevention of generation of dewdrops and prevention of penetration of water drops into the test chamber.

A light source chamber 14 having a light source 13 isolated by a separating plate 12 is provided on the top of the test chamber 3. The light source chamber 14 is cooled by a cooling unit 15 for a light source cooling system and a blower 16 for the light source cooling system so as not to have an influence of heat from the light source 13 on the test chamber 3.

In addition, these respective instruments are so constructed that they can be suitably controlled by controlling means 17. An inlet for an environmental gas supplied to the test chamber 3, and measuring instruments 18 for detecting the temperature and humidity of the atmosphere supplied are provided inside the test chamber. Signals based on information measured by the measuring instruments 18 are sent to the controlling means 17, whereby the temperature and humidity can be exactly controlled.

An image used in the present invention may be any of an image by ink-jet recording, an image by electrophotographic recording, an image by thermal ink-transfer recording, an image by thermal color recording and a silver halide photograph. However, the present invention is particularly preferably applied to an image recorded by ink compositions, for example, an image formed by an ink-jet recording method.

The compositions of an ink composition and a recording medium used may not be limited so far as they are suitable for the printing method thereof. The composition of a representative ink used will hereinafter be described.

### (Aqueous medium)

An aqueous medium contained in an ink is composed of a mixed solvent of water and a water-soluble organic solvent. A solvent having an anti-drying effect on an ink is particularly preferred as the water-soluble organic solvent. As the water, it is desirable to use deionized water instead of tap water containing various ions.

Specific examples of the water-soluble organic solvent used in the present invention include alkyl alcohols having 1 to 5 carbon atoms, such as methyl alcohol, ethyl alcohol, n-propyl alcohol, isopropyl alcohol, n-butyl alcohol, sec-butyl alcohol, tert-butyl alcohol, isobutyl alcohol and n-pentanol; amides such as dimethylformamide and dimethylacetamide; ketones and keto-alcohols such as acetone and diacetone alcohol; ethers such as tetrahydrofuran and dioxane; oxyethylene or oxypropylene copolymers such as diethylene glycol, triethylene glycol, tetraethylene glycol, dipropylene glycol, tripropylene glycol, polyethylene glycol and polypropylene glycol; alkylene glycols the alkylene moiety of which has 2 to 6 carbon atoms, such as ethylene glycol, propylene glycol, trimethylene glycol and triethylene glycol; 1,2,6-hexanetriol; glycerol; lower alkyl ethers such as ethylene glycol monomethyl (or monoethyl) ether, diethylene glycol monomethyl (or monoethyl) ether and triethylene glycol monomethyl (or monoethyl) ether; lower dialkyl ethers of polyhydric alcohols, such as triethylene glycol dimethyl (or diethyl) ether and tetraethylene glycol dimethyl (or diethyl) ether; alkanolamines such as monoethanolamine, diethanolamine and triethanolamine; sulfolane; N-methyl-2-pyrrolidone; 2-pyrrolidone; and 1,3-dimethyl-2-imidazolidinone. Such water-soluble organic solvents as mentioned above may be used either singly or in any combination thereof. As the water that is a main solvent for the ink composition, may be used purified water such as ion-exchanged water, ultrafiltered water, reverse-osmosed water or distilled water, or superpurified water. Water sterilized by irradiation of ultraviolet light or the like may preferably be used to prevent occurrence of mildew and bacteria when the ink composition is stored for a long period of time.

No particular limitation is imposed on the content of such water-soluble organic solvents as described above. However, it is preferably within a range of from 3 to 50% by mass based on the total mass of the ink. The content of water contained in the ink is preferably within a range of from 50 to 95% by mass based on the total mass of the ink. Besides the above components, a viscosity modifier, an antifoaming agent, an antiseptic, a mildewproofing agent, a surfactant, an antioxidant and/or the like may be added to the ink to obtain an ink having desired physical properties.

### (Coloring material)

A coloring material used in the ink composition may be either a dye or a pigment.

An organic pigment or inorganic pigment may be used as the pigment without any particular limitation. As the organic pigments, may be used azo pigments (including azo lake, insoluble azo pigments, fused azo pigments, chelated azo pigments, etc.), polycyclic pigments (for example, perylene pigments, phthalocyanine pigments, anthraquinone pigments, quinacridone pigments, dioxazine pigments, thioindigo pigments, isoindolinone pigments and quinophthalone pigments), dye chelates (basic dye type chelates, acid dye type chelates, etc.), nitro pigments, nitroso pigments, aniline black or the like. As the inorganic pigments, may be used titanium oxide and iron oxide, and besides carbon black produced by a publicly known, process such as the contact process, furnace process or thermal process, or the like.

As the dyes, various kinds of dyes used in ink-jet recording, such as direct dyes, acid dyes, food colors, basic dyes, reactive dyes, disperse dyes and reactive disperse dyes, may be used without any particular limitation.

### (Recording medium)

No particular limitation is also imposed on the recording medium because it is suitably selected according to the recording system used. Examples of the recording medium applicable to an ink-jet recording method include paper, films, fibers and leather. Those obtained by providing an ink-receiving layer on these base materials may also be used. The ink-receiving layer may be provided by, for example, impregnating or coating such a base material as described above with a cationic polymer, or coating the surface of the base material with porous silica, alumina sol or special ceramic together with a hydrophilic polymer such as polyvinyl alcohol.

### (Recording method)

As the recording method, may be used any system such as an ink-jet recording system making good use of bubbles caused by heating or mechanical vibration.

The present invention will hereinafter be described in more detail by Examples.

### EXAMPLE 1:

Measuring conditions of an apparatus for weatherability test were set as follows.
Temperature within a test chamber: 24°C
Relative humidity within the test chamber: 60% RH
Gas composition: O₃ 1,200 ppb, NO₂ 1,250 ppb, SO₂ 300 ppb, H₂S 50 ppb and Cl₂ 10 ppb
Supply system of mixed gas: constant flow rate gas flow system (flow rate: 1,000 liters/h, flow velocity: 0.5 m/s)
Light source: white fluorescent lamp
Illuminance on the surface of an image sample: 25,000 lux

### Test time: 72 hours.

A print by an ink-jet printer was used as an image sample. BJ-F 900 (trade name, manufactured by Canon Inc.) as a printer, genuine paper: Professional Photopaper (PR-101, trade name) as paper for printing and an ink cartridge set BCI-6 series (trade name, cyan, magenta, yellow, photocyan and photomagenta) as inks were used. As an image pattern, was used a high-definition color digital standard image data (ISO/JIS-SCID, according to JIS X 9201-1995).

With respect to a background portion, a hair portion and a skin portion in an N1 portrait of the resulting image as well as color patches of cyan C, magenta M yellow Y, black Bk and process black PCBk (black obtained by mixing cyan, magenta and black), a color difference prescribed by CIE L*a*b* was measured, and the retention ((optical density after test/optical density before test) × 100 (%)) of the optical density (OD) for each color patch was determined to find differences (ΔE and ΔOD) from a sample exhibited (for a year) in an actual environment. Spectrolino (trade name, manufactured by GRETAG Co.) was used for measurement of the color differences and optical densities. The results are shown in Table 1. The results of the retention of the optical densities for other color patches than PCBk are shown in Table 2.

### COMPARATIVE EXAMPLE 1:

An image sample was evaluated in the same manner as in EXAMPLE 1 except that the gas composition was changed to ozone alone (1,200 ppb). The results are shown in Tables 1 and 2.

### COMPARATIVE EXAMPLE 2:

An image sample was evaluated in the same manner as in EXAMPLE 1 except that the gas composition was changed to sulfur dioxide alone (300 ppb). The results of the retention (%) of the optical densities for the color patches are shown in Table 2.

### COMPARATIVE EXAMPLE 3:

An image sample was evaluated in the same manner as in EXAMPLE 1 except that the gas composition was changed to hydrogen sulfide alone (300 ppb). The results of the retention (%) of the optical densities about the color patches are shown in Table 2.

### COMPARATIVE EXAMPLE 4:

An image sample was evaluated in the same manner as in EXAMPLE 1 except that no mixed gas was used. The results of the retention (%) of the optical densities for the color patches are shown in Table 2.

### COMPARATIVE EXAMPLE 5:

An image sample was evaluated in the same manner as in EXAMPLE 1 except that no light was applied. The results of the retention (%) of the optical densities for the color patches are shown in Table 2.

**Table 1**

| | | EXAMPLE 1 | | COMPARATIVE EXAMPLE 1 | |
|---|---|---|---|---|---|
| | | ΔE | ΔOD (%) | ΔE | ΔOD (%) |
| Portrait | Background | 2 | - | 15 | - |
| | Skin portion | 2 | - | 2 | - |
| | Hair portion | 2 | - | 2 | - |
| Color patch | C | 3 | 3 | 5 | 15 |
| | M | 1 | 1 | 5 | 35 |
| | Y | 1 | 1 | 5 | 3 |
| | Bk | 2 | 3 | 10 | 10 |
| | PCBk | 3 | - | 10 | - |

**Table 2**

| | C | M | Y | Bk |
|---|---|---|---|---|
| Actual environment | 82 | 93 | 93 | 58 |
| EXAMPLE 1 | 85 | 92 | 92 | 55 |
| COMPARATIVE EXAMPLE 1 | 67 | 58 | 90 | 48 |
| COMPARATIVE EXAMPLE 2 | 97 | 90 | 95 | 94 |
| COMPARATIVE EXAMPLE 3 | 97 | 90 | 95 | 94 |
| COMPARATIVE EXAMPLE 4 | 99 | 91 | 96 | 95 |
| COMPARATIVE EXAMPLE 5 | 87 | 98 | 98 | 63 |

As apparent from the results described above, it is revealed that the test performed in accordance with the testing apparatus used in and the testing process according to the present invention more closely simulate the color fading in the actual environment when the results of the accelerated test by the apparatus for weatherability test of images according to the present invention are compared with the results of the conventional test with a single gas (ozone, sulfur dioxide or hydrogen sulfide) as well as the results of the complex exposure by a single gas (ozone, sulfur dioxide or hydrogen sulfide) and light (fluorescent lamp). It is also revealed that differences between them are extremely marked at portions conspicuous in discoloration, such as colors in the background portion, PCBk and Bk.

## Claims

1. A testing process for evaluating the indoor weatherability of an ink-jet recorded image, comprising the steps of
placing the image in a predetermined atmosphere in a test chamber, and
irradiating the image in said atmosphere with a predetermined quantity of light having a predetermined spectral distribution from a light source chamber with a fluorescent lamp,
**characterized in that:**
said predetermined atmosphere is formed by a mixed gas containing 100 to 3,000 ppb of ozone, 100 to 3,000 ppb of nitrogen dioxide, 25 to 900 ppb of sulfur dioxide, 1 to 150 ppb of hydrogen sulfide, and 1 to 30 ppb of chlorine; and
an influence of heat due to the light irradiation on the mixed gas atmosphere is reduced by cooling the fluorescent lamp and by providing a separating plate that isolates the light source chamber from the test chamber.

2. The process according to claim 1, wherein the mixed gas is continuously supplied into the test chamber during the weatherability test.

3. The process according to claim 2, wherein the mixed gas is supplied with a flow rate of 500 to 5,000 liters/h.

4. The process according to claim 2, wherein the mixed gas is supplied with a flow velocity of 0.1 to 2.5 m/s.

5. The process according to any one of claims 1 to 4, wherein the predetermined atmosphere has a predetermined temperature and a predetermined humidity.

## Patentansprüche

1. Prüfverfahren zum Ermitteln der Innenraum-Klimabeständigkeit eines tintenstrahlaufgezeichneten Bildes, umfassend die Schritte:
Platzieren des Bildes in einer vorbestimmten Atmosphäre in einer Prüfkammer und
Bestrahlen des Bildes in der Atmosphäre mit einer vorbestimmten Lichtmenge einer vorbestimmten Spektralverteilung mit Hilfe einer Lichtquelle, die eine Fluoreszenzlampe beinhaltet,
**dadurch gekennzeichnet, dass**
die vorbestimmte Atmosphäre gebildet wird durch ein Gasgemisch, enthaltend 100 bis 3000 ppb (parts per billion = Teile pro Milliarde) Ozon, 100 bis 3000 ppb Stickstoffdioxid, 25 bis 900 ppb Schwefeldioxid, 1 bis 150 ppb Schwefelwasserstoff und 1 bis 30 ppb Chlor; und
ein Wärme-Einfluss wegen der Lichteinstrahlung auf die Gasgemisch-Atmosphäre verringert wird durch Kühlen der Fluoreszenzlampe und durch Vorsehen einer Separierplatte, die die Lichtquellenkammer von der Prüfkammer entkoppelt.

2. Verfahren nach Anspruch 1, wobei
während der Klimabeständigkeitsprüfung das Gasgemisch der Prüfkammer kontinuierlich zugeführt wird.

3. Verfahren nach Anspruch 2, wobei
das Gasgemisch mit einem Durchsatz von 500 bis 5000 Liter/h zugeführt wird.

4. Verfahren nach Anspruch 2, wobei
das Gasgemisch mit einer Strömungsgeschwindigkeit von 0,1 bis 2,5 m/s zugeführt wird.

5. Verfahren nach einem der Ansprüche 1 bis 4, wobei
die vorbestimmte Atmosphäre eine vorbestimmte Temperatur und eine vorbestimmte Feuchtigkeit besitzt.

## Revendications

1. Procédé d'essai pour évaluer la tenue aux conditions ambiantes intérieures d'une image enregistrée par jet d'encre, comprenant les étapes qui consistent :
à placer l'image dans une atmosphère prédéterminée dans une chambre d'essai, et
à irradier l'image dans ladite atmosphère avec une quantité de lumière prédéterminée ayant une distribution spectrale prédéterminée depuis une chambre de source de lumière avec une lampe à fluorescence,
**caractérisé en ce que :**
ladite atmosphère prédéterminée est formée par un mélange gazeux contenant 100 à 3000 ppb d'ozone, 100 à 3000 ppb de dioxyde d'azote, 25 à 900 ppb d'anhydride sulfureux, 1 à 150 ppb d'hydrogène sulfuré et 1 à 30 ppb de chlore ; et
l'effet de la chaleur dû à l'irradiation par la lumière sur l'atmosphère constituée du mélange gazeux est réduit en refroidissant la lampe à fluorescence et en plaçant une plaque séparée qui isole la chambre à source de lumière de la chambre d'essai.

2. Procédé selon la revendication 1, dans lequel le mélange gazeux est fourni en continu dans la chambre d'essai pendant l'essai de tenue aux conditions ambiantes.

3. Procédé selon la revendication 2, dans lequel le mélange gazeux est fourni à un débit d'écoulement de 500 à 5000 litres/heure.

4. Procédé selon la revendication 2, dans lequel le mélange gazeux est fourni à une vitesse d'écoulement de 0,1 à 2,5 mètres/seconde.

5. Procédé selon l'une quelconque des revendications 1 à 4, dans lequel l'atmosphère prédéterminée a une température prédéterminée et une humidité prédéterminée.
